(19) Europäisches Patentamt
European Patent Office
Office européen des brevets

(11) **EP 2 891 747 B1**

(12) **EUROPEAN PATENT SPECIFICATION**

(45) Date of publication and mention
of the grant of the patent:
**08.08.2018  Bulletin 2018/32**

(51) Int Cl.:
*D21B 1/32* *(2006.01)*    *A61F 5/44* *(2006.01)*
*B09B 5/00* *(2006.01)*    *C08J 11/06* *(2006.01)*
*B09B 3/00* *(2006.01)*    *A61F 13/15* *(2006.01)*

(21) Application number: **13813071.1**

(22) Date of filing: **25.06.2013**

(86) International application number:
**PCT/JP2013/067356**

(87) International publication number:
**WO 2014/007105 (09.01.2014 Gazette 2014/02)**

(54) **RECYCLED FIBER AND PROCESS FOR PREPARING THE RECYCLED FIBER**

WIEDERVERWERTETE FASER UND VERFAHREN ZUR HERSTELLUNG DER
WIEDERVERWERTETEN FASER

FIBRE RECYCLÉE ET PROCÉDÉ DE PREPARATION DE FIBRE RECYCLÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **06.07.2012   JP 2012152879**

(43) Date of publication of application:
**08.07.2015   Bulletin 2015/28**

(73) Proprietor: **Nippon Paper Industries Co., Ltd.
Tokyo 114-0002 (JP)**

(72) Inventors:
• **WATANABE, Harutaka
Tokyo 114-0002 (JP)**
• **NAKATANI, Toru
Tokyo 114-0002 (JP)**
• **GOTO, Shisei
Tokyo 114-0002 (JP)**
• **KATORI, Eisaku
Fukuchiyama-shi, Kyoto 620-0853 (JP)**
• **TAKAHASHI, Tetsuro
Tokyo 101-00662 (JP)**
• **HAYASAKA, Hideki
Tokyo 101-00662 (JP)**

• **The other inventors have waived their right to be
thus mentioned.**

(74) Representative: **Carpmaels & Ransford LLP
One Southampton Row
London WC1B 5HA (GB)**

(56) References cited:
EP-A1- 0 558 512      EP-A1- 1 081 285
JP-A- H06 502 454     JP-A- 2000 084 533
JP-A- 2001 310 178    JP-A- 2003 135 521
JP-A- 2003 225 645    JP-A- 2004 009 007
JP-A- 2004 082 700    JP-A- 2010 059 586
JP-A- 2012 081 433    US-A1- 2005 220 542

• N N: "From Waste to Profitable Material Recovery
of cellulose fibers and superabsorbents improve
profitability", INTERNET CITATION, 12 March
2007 (2007-03-12), pages 1-4, XP002700101,
Retrieved from the Internet:
URL:http://www.venti-oelde.com/uploads/tx_
sbdownloader/sap_rueckgewin-en.pdf
[retrieved on 2013-07-04]

**Description**

TECHNICAL FIELD

The present invention relates to recycled fibers obtained from sanitary products containing cellulose pulp by recycling processes, a recycled fiber molding comprising a recycled fiber, and to a process for preparing the recycled fiber.

BACKGROUND ART

Sanitary products such as disposable paper diapers typically consist of the following components: an absorbent core made of a pulp fiber or the like; a SAP (superabsorbent high molecular polymer) retaining water absorbed by the absorbent core; and an enveloping material made of a nonwoven fabric, plastic or the like for enveloping them. These sanitary products have been disposed of and burnt rather than reused multiple times, but recent environmental awareness has led to a growth in the necessity of recovering and recycling these components.

For example, patent document 1 relates to recycled diapers and recycled incontinence pads using recycled pulp obtained from used diapers or used incontinence pads, and describes that they are prepared by crushing used diapers or used incontinence pads consisting of a fluff pulp, a high molecular absorbent polymer, a nonwoven fabric and a waterproof sheet and dissolving the resulting fragments in water containing a water-releasing agent in an agitator to release water absorbed by the high molecular absorbent polymer; removing the nonwoven fabric and the waterproof sheet in a circulating separator to recover the pulp component; further separating the pulp component in a gravity separator to recover an upper layer pulp component; subjecting the resulting pulp component to washing, microbicidal and drying processes to give a recycled fluff pulp; and forming the recycled fluff pulp obtained through the foregoing steps into a sheet.

Patent document 2 relates to processes for separating and recovering pulp components and absorbent polymers from used absorbent articles, and describes adding a transition metal salt alone or a mixture of a transition metal salt and an alkali metal salt or an alkaline earth metal salt to a gelled mixture of pulp components and absorbent polymers contained in a used absorbent article, thereby removing water contained in the absorbent polymers to contract and solidify the absorbent polymers and staining the absorbent polymers with the transition metal salt; and then separating and recovering the pulp components and the absorbent polymers.

Further, patent document 3 describes a process comprising treating a women's menstrual paper product containing a superabsorbent polymer in at least one bath of an aqueous solution to dissolve soluble matters from the product; and treating the superabsorbent polymer with at least one water-soluble compound of an alkali metal, an alkaline earth metal, aluminum, copper (II), iron (III) and zinc to reduce swelling of the superabsorbent polymer in the aqueous solution.

In addition, patent documents 4 to 7 report techniques for recovering components from used sanitary products, and patent document 8 reports a technique for preparing fuels such as RPF from used sanitary products. Patent document 9 (JPA 2000-084533) discloses a method of separating and recovering a pulp component in a spent paper diaper for reutilization, to reduce environmental destruction such as deforestation for securing pulp resource and to decrease the generation of pollution caused by the incineration. Patent document 10 discloses a process for the treatment of absorbent sanitary paper products to separate such products into components thereof. Patent document 11 discloses a process to sanitarily recover material from a soiled sanitary article by disinfecting or sterilizing colibacillus or other various germs which may adhere to the soiled sanitary article containing high water-absorbent, gelatinous resin on absorbing water. Patent document 12 discloses a landfill cover composition comprising a concentrate formulated to include a hardening agent, a thickener and emulsifier, and a super absorbent polymer, mixable with water and a filler material, such as cellulose or wood fiber mulch. Patent document 13 discloses a method for producing a pulp molded article. Non-patent document 14 discloses manufacture of hygienic products and reuse of waste.

CITATION LIST

PATENT LITERATURE

Patent document 1: JPA 2003-135521
Patent document 2: JPA 2003-225645
Patent document 3: JPA-H6-502454
Patent document 4: JPA 2001-310178
Patent document 5: JPA 2003-200147
Patent document 6: JPA 2004-042038

Patent document 7: JPA 2004-008270
Patent document 8: JPA 2006-007111
Patent document 9: JPA 2000-084533
Patent document 10: EP 0 558 512 A1
Patent document 11: JP 2004 082700
Patent document 12: US 2005/220542 A1
Patent document 13: EP 1 081 285 A1
Non-patent document 14: "From Waste to Profitable Material; Recovery of Cellulose Fibers and Superabsorbents Improve Profitability" (http://www.venti-oelde.com/uploads/tx_sbdownloader/sap_rueckgewin-ed.pdf).

## SUMMARY OF INVENTION

### TECHNICAL PROBLEM

As described above, some methods for recovering and recycling components from used sanitary products have been proposed. However, the prior techniques had the disadvantage that SAPs and nonwoven fabrics are mixed into the recycled pulp fibers and deposited on tools during the preparation of moldings from the recycled fibers, thus impairing the runnability. Further, the recycled fibers currently remain in limited fields of application such as construction materials because of not only their own poor appearance but also poor appearance of moldings using the recycled fibers, as well as insufficient strength of such moldings and other disadvantages.

Thus, the present invention aims to provide recycled fiber moldings having a low dirt content and excellent appearance and strength properties.

### SOLUTION TO PROBLEM

We found that the problems described above can be solved by recycled fibers characterized by a low SAP content in contrast to those recycled by conventional methods.

The present invention includes, but not limited to, the following.

(1) A recycled fiber obtained by subjecting a sanitary product containing a cellulose pulp to a recycling process, which has a superabsorbent polymer (SAP) content of less than 5 %, a Canadian standard freeness of 650 ml or more when determined according to JIS P 8121:1995, a length weighted average fiber length of 0.7 mm or more, and a dirt area fraction of 200 $mm^2/m^2$ or less when determined by a dirt assay on a handsheet having a basis weight of 60 $g/m^2$ prepared according to JIS P 8209.

(2) The recycled fiber as defined in (1), which has a dirt area fraction of 50,000 $mm^2/m^2$ or less when determined by a staining assay using cobalt (II) chloride hexahydrate on a handsheet having a basis weight of 60 $g/m^2$ prepared according to JIS P 8209.

(3) The recycled fiber as defined in (1) or (2), which has a superabsorbent polymer content of 1 % or less.

(4) The recycled fiber as defined in any one of (1) to (3), which has a dirt area fraction of 1,000 $mm^2/m^2$ or less when determined by a staining assay using cobalt (II) chloride hexahydrate on a handsheet having a basis weight of 60 $g/m^2$ prepared according to JIS P 8209.

(5) The recycled fiber as defined in any one of (1) to (4) for use in construction materials, paperboards, cushioning materials or molded products.

(6) The recycled fiber as defined in (3) or (4) for use in sanitary products, fluff pulps, writing and printing papers or tissue papers.

(7) A slurry comprising the recycled fiber as defined in any one of (1) to (6).

(8) A wet pulp comprising the recycled fiber as defined in any one of (1) to (6).

(9) A dry pulp comprising the recycled fiber as defined in any one of (1) to (6).

(10) A recycled fiber molding comprising a recycled fiber as defined in (1).

(11) A process for preparing the recycled fiber as defined in any one of (1) to (6), comprising at least the steps of disintegrating a used sanitary product and dispersing it in water, and separating and recovering fibers and SAPs contained in the sanitary product, characterized in that the step of disintegrating a sanitary product and dispersing it in water comprises adding a bleaching/microbicidal agent, a crosslinking agent and an acidic agent as additives; wherein the step of disintegrating a sanitary product and dispersing it in water comprises disintegration at a consistency of 3 to 20 % in the presence of sodium hypochlorite as a bleaching/microbicidal agent and calcium chloride as a crosslinking agent, followed by dilution to a consistency of 2 % or less and then adding an acidic agent to adjust the pH at 4.5 to 5.5, and also comprises heating at one or more points of the step; and wherein the separation/recovery step comprises using a cascade separator or feed forward separator having two or more stages.

ADVANTAGEOUS EFFECTS OF INVENTION

Recycled fiber moldings having a low dirt content and excellent appearance and strength properties can be obtained by using the the recycled fibers of the present invention having a low SAP content.

BRIEF DESCRIPTION OF DRAWINGS

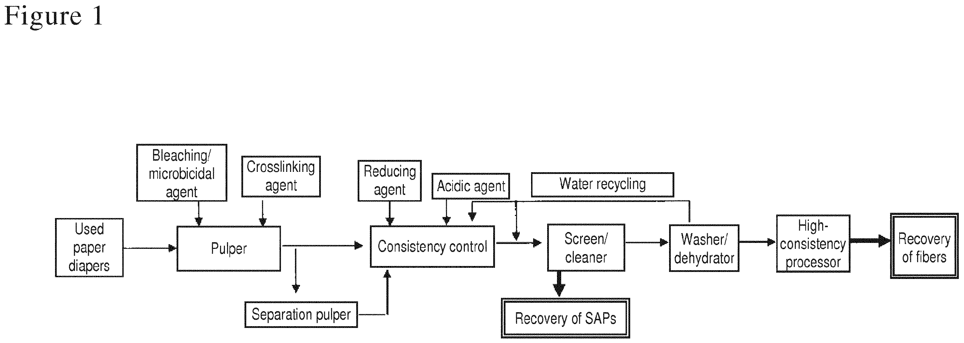

Figure 1 is a flow diagram showing one embodiment of a process of the present invention.

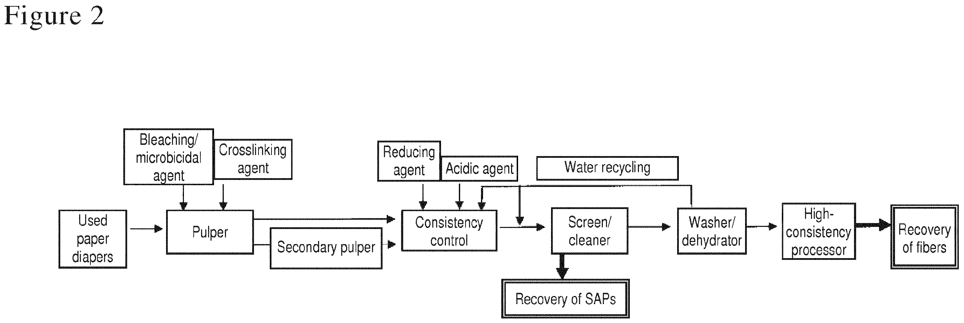

Figure 2 is a flow diagram showing one embodiment of a process of the present invention.

DESCRIPTION OF EMBODIMENTS

In the present invention, sanitary products from which the recycled fibers are derived include disposable paper diapers, incontinence products, women's menstrual products, bed pads and the like, but they are not limited to these examples so far as they contain a cellulose pulp (fiber). The sanitary products may be unused defective products discarded on reject lines of factories and the like or used products collected by hospitals, communities and the like. It should be noted that paper diapers typically consist of the following components: an absorbent core made of a pulp fiber or the like; a SAP (superabsorbent high molecular polymer) retaining water absorbed by the absorbent core; and an enveloping material made of a nonwoven fabric, plastic or the like for enveloping them.

In the present invention, the recycled fibers comprise a cellulose pulp as a main component as well as a superabsorbent polymer (SAP) and a synthetic fiber (polypropylene, polyethylene or the like) such as a plastic or nonwoven fabric contained as components of wrapping bags and paper diapers. The cellulose pulp is a fiber containing a cellulose as a main component derived from a wood or non-wood plant; and examples of wood pulps include chemical pulp fibers such as softwood and hardwood kraft pulp and sulfite pulp; mechanical pulp fibers such as softwood and hardwood groundwood pulp, refiner groundwood pulp, thermomechanical pulp and chemithermomechanical pulp; and recycled pulp fibers derived from sheet-like materials consisting of waste paper or cellulose and the like; while examples of non-wood plant-derived fibers include fibers of cotton, hemp, kenaf, straw, reed, mulberry, cotton tree, sugar cane, *Broussonetia kazinoki* × *B. papyrifera, Edgeworthia chrysantha* and the like. Regenerated cellulose fibers such as rayon are also included. The "SAP" is a superabsorbent high molecular polymer, examples of which include sodium polyacrylate, carboxymethyl cellulose (CMC), polyvinyl alcohol (PVA), PVA/sodium polyacrylate and the like. The most commonly used is crosslinked sodium polyacrylate. Further, the nonwoven fabric is a sheet-like material made of fibers, which are bonded together by heat or an adhesive or intertwined with each other rather than woven, and these fibers include synthetic fibers such as polypropylene and polyester; natural fibers such as wool and cotton; chemical fibers such as rayon and acetate fibers; inorganic fibers such as glass fibers and carbon fibers and the like.

The recycled fibers according to the present invention are characterized by a SAP content of less than 5 %, more preferably less than 3 %, even more preferably less than 1 %. The SAP content refers to the value determined by the composition analysis of starting materials by an enzymatic assay ("Quantification of pulp and polymer absorbents in recycling processes of paper diapers", by Ikeura et al., in the Journal of Environmental Laboratories Association vol. 36, No. 1, p 51-p 58 (2011)). If the SAP content exceeds 10 %, SAPs will be deposited in quantity on tools or the like during the use of the recycled fibers, resulting in not only a significant decrease in productivity during the preparation of moldings from the recycled fibers but also a poor appearance of the moldings. Moreover, SAPs have the property of retaining water so that higher SAP contents are inconvenient because dehydration or drying is impaired to cause problems such as insufficient drying, low productivity and even low product strength short of the design strength and the like. As an indicator of water retention of recycled fibers, freeness can be applied, which measures the water retention or dehydration of pulp slurries. Especially, fluff pulps used for paper diapers and the like are required to have a very high Canadian standard freeness (CSF). The recycled fibers according to the present invention have a CSF of 650 ml or more, preferably 700 ml or more. For uses in recycled paper or other applications requiring strength, the recycled fibers obtained can be treated by a beater such as a refiner or a high pressure homogenizer or the like to lower the CSF to a desired value.

As an indirect indicator of the strength of recycled fibers, fiber length can be applied. If the average fiber length is too short, it is difficult to attain a desired strength. The recycled fibers according to the present invention have a length weighted average fiber length of 0.7 mm or more, preferably 1.5 mm or more, more preferably 1.8 mm or more, especially preferably 2.0 mm or more.

As one of indicators of the appearance of moldings, an evaluation method based on the dirt area on a sheet can be applied. If the dirt area is too large, numerous lumps of SAPs are observed on the fiber surfaces, which greatly impair not only appearance but also surface smoothness or aesthetics. Preferably, the recycled fibers according to the present invention have a dirt area fraction of 50,000 $mm^2/m^2$ (or 100,000 dirt particles/$m^2$) or less, more preferably 40,000 $mm^2/m^2$ or less, even more preferably 20,000 $mm^2/m^2$ or less, especially preferably 10,000 $mm^2/m^2$ or less when determined by a staining assay using cobalt (II) chloride hexahydrate. When the recycled fibers are used to prepare moldings, the dirt area fraction is most preferably 1,000 $mm^2/m^2$ or less especially for applications requiring attractive appearance such as paper products and recycled diapers. It should be noted that this assay detects all of three types of dirt particles, i.e., white, transparent and colored dirt particles because white and transparent dirt particles are also stained. SAPs are white or light yellow so that they could not be sometimes detected by conventional dirt assays. However, any SAPs that may adversely affect not only appearance but also strength can be quantified by using a staining assay.

In the recycled fibers of the present invention, the dirt area fraction determined by a dirt assay on a handsheet having basis weight of 60g/$m^2$ prepared according to JISP8209 is 200 $mm^2/m^2$ or less. This assay detects originally colored ones of dirt particles. Dirt particles that can be detected by this assay are plastics contained in sanitary products, deposited soils, contaminants brought in during the recycling process and the like. This means that dirt particles which are readily identifiable by visual inspection can be quantified, thus providing an indicator especially for evaluation of appearance.

In the present invention, the recycled fibers may be obtained by any method, but desirably using a recycling process combining a chemical treatment to modify properties of SAPs and a cleaning system. For example, they can be obtained by combining a method for modifying properties of SAPs with a crosslinking agent such as calcium chloride or an acidic chemical such as sulfuric acid and a method for removing dirt particles by using a cleaning system such as a cleaner or a screen, as disclosed in our Japanese Patent Publication Nos. 2012-287476 and 2012-287496.

Further, the recycled fibers of the present invention can be subjected to a sterilization or microbicidal treatment. Specifically, the recycled fibers of the present invention can be subjected to a sterilization or microbicidal treatment using, for example, ozone, sodium hypochlorite, ethylene oxide gas (EOG), heating, autoclaving or other means.

The recycled fibers in the present invention can be provided in the form of a slurry, wet pulp or dry pulp as a material for preparing moldings. The term "slurry" refers to a flowable state having a solids content of about 10 % or less, while the term "wet pulp" refers to a state having a solids content of about 10 to 50 %, preferably about 30 to 50 % and positioned between slurry and dry pulp. It should be noted that the solids contents shown above are merely typical ranges, but the present invention is not limited to these values.

Moldings incorporating the recycled fibers include, but not limited to, fluff pulps, paper diapers, construction materials (ceramic siding applications, exterior wall materials, interior wall materials), papers (newsprint paper, coated paper, recycled paper and the like), paperboards (liners, cores), facial tissues, toilet rolls, paper wipers, cushioning materials, molded products, reinforced plastics and the like. When the recycled fibers are used for these moldings, various quality requirements for the recycled fibers applied as starting materials including purity, strength, dirt content, the dirt area fraction of a sheet formed therefrom as well as processability into an end product can be appropriately determined depending on the purpose and application.

The recycled fibers according to the present invention have a superabsorbent polymer content as low as less than 5 % so that they are suitable for use in not only sanitary products such as paper diapers, fluff pulps, writing and printing papers, or tissue papers such as facial tissue but also construction materials, paperboards, cushioning materials or molded products. Especially when the superabsorbent polymer content is 1 % or less, the recycled fibers of the present invention can be conveniently used in sanitary products such as paper diapers, fluff pulps, writing and printing papers, or tissue papers such as facial tissue.

Further, the recycled fibers according to the present invention are suitable for use in not only sanitary products such as paper diapers, fluff pulps, writing and printing papers, or tissue papers such as facial tissue but also construction materials, paperboards, cushioning materials or molded products when they have a dirt area fraction of 50000 $mm^2/m^2$ or less as determined by a staining assay using cobalt (II) chloride hexahydrate on a handsheet having a basis weight of 60 g/$m^2$ prepared according to JIS P 8209. Especially when the dirt area fraction is 1000 $mm^2/m^2$ or less, the recycled fibers of the present invention can be conveniently used in sanitary products such as paper diapers, fluff pulps, writing and printing papers, or tissue papers such as facial tissue.

Processes for preparing the recycled fibers

In preferred embodiments, the recycled fibers according to the present invention can be prepared by the treating processes described below. According to the invention, the step of disintegrating a used sanitary product and dispersing it in water comprises adding a bleaching/ microbicidal agent, a crosslinking agent and an acidic agent because the efficiency of separating and recovering components can be increased. The following clauses (1) to (12) do not fall under the scope of the claims.

(1) A process for treating a used sanitary product, comprising at least the steps of disintegrating the sanitary product and dispersing it in water; and separating and recovering fibers and SAPs contained in the sanitary product, wherein the step of disintegrating the sanitary product and dispersing it in water comprises adding a crosslinking agent and an acidic agent.

(2) The process as defined in (1) wherein the disintegration/dispersion step further comprises adding a bleaching/microbicidal agent.

(3) The process as defined in (1) or (2) wherein disintegration takes place at a consistency of 3.0 to 20.0 % in the presence of calcium chloride as a crosslinking agent, followed by adding an acidic agent.

(4) The process as defined in any one of (1) to (3) wherein disintegration is followed by dilution to a slurry consistency of 2 % or less, and then adding an acidic agent.

(5) The process as defined in any one of (1) to (4) wherein the acidic agent is added to adjust the pH of the slurry at 6 or less.

(6) The process as defined in any one of (1) to (5) comprising adding a reducing agent prior to adding an acidic agent.

(7) The process as defined in any one of (1) to (6) comprising heating at one or more points of the step.

(8) The process as defined in any one of (1) to (7) wherein a tub pulper is used for disintegrating the sanitary product.

(9) The process as defined in (8) wherein the tub pulper is immediately followed by a separation pulper and/or a detrasher.

(10) The process as defined in (8) or (9) wherein the tub pulper is provided with a rope ragger.

(11) The process as defined in any one of (1) to (10) wherein the separation/recovery step comprises using a screen and/or a cleaner.

(12) The process as defined in any one of (1) to (11) wherein the separation/recovery step comprises washing and dehydrating a dispersion containing fibers using a vertical washer, and using the removed water as process water.

## 1. Components

As used herein, the "sanitary product" comprises a fiber and a SAP (superabsorbent polymer), examples of which include, but not limited to, disposable paper diapers, incontinence products, women's menstrual products, bed pads and the like. The present invention will be sometimes described below by taking paper diapers as examples, but the present invention is not limited to them.

As used herein, the "fiber" means to include pulp fibers and synthetic fibers (polypropylene, polyethylene and the like) such as nonwoven fabrics among components of sanitary products such as paper diapers. The SAP refers to a superabsorbent polymer, examples of which include sodium polyacrylate, carboxymethyl cellulose (CMC), polyvinyl alcohol (PVA), PVA/sodium polyacrylate and the like. The most commonly used is crosslinked sodium polyacrylate.

## 2. Process flows and systems

<Process flows>

For example, the following process flows can be applied for treating used paper diapers depending on the desired quality and process cost.

In the separation/recovery step according to the present invention, components such as fibers and SAPs are recovered, among which fibers can be collected as accepts through a cleaner or a screen or the like, for example, while components other than fibers (SAPs and the like) can be collected as rejects. It should be noted that the components other than fibers may be herein sometimes referred to as contaminants and that components such as SAPs can be further separated and recovered from contaminants (components other than fibers) collected as rejects.

- Flow 1 (low quality/low cost):
  Disintegration → cleaner and/or screen → dehydrator/concentrator → washer.
- Flow 2 (medium quality/medium cost):
  Disintegration → cleaner and/or screen → dehydrator/concentrator → high-consistency processor → washer.
- Flow 3 (high quality/high cost):
  Disintegration → coarse screen → cleaner → dehydrator/concentrator → high-consistency processor → fine screen → washer/dehydrator.
- Flow 4 (high quality/high cost):
  Disintegration → dehydrator/concentrator → high-consistency processor → coarse screen → cleaner → fine screen → washer/dehydrator.

The drawings illustrate specific examples of process flows, but the present invention is not limited to these examples. In the drawings, the first step of disintegrating a sanitary product and dispersing it in water involves feeding used paper diapers as starting materials into a pulper and dispersing them in water. During then, a bleaching/microbicidal agent and a crosslinking agent are added into the pulper. Then, the dispersion is diluted to control the consistency, and a reducing agent and an acidic agent are successively added to adjust the pH. The next step of separating and recovering fibers and SAPs contained in the paper diapers involves treating the dispersion using a screen and a cleaner to recover SAPs first. On the other hand, the dispersion containing fibers is then washed/dehydrated by a dehydrator or the like. The water removed here can be returned to the process line and reused. Further, the dispersion containing fibers is treated by a concentrator. Then, it is optionally washed/dehydrated and screened to recover fibers, though not shown in the drawings. In addition to fibers, plastics contained in nonwoven fabrics or the like are also separated and recovered, though not shown in the drawings.

<Pulper>

In the present invention, a pulper is preferably employed as a machine for disintegrating used paper diapers and dispersing them in water. The pulper is suitably the one employed for disintegrating waste paper, preferably a system consisting of a low-consistency pulper or a vertical batch high-consistency tub pulper and a subsequent secondary pulper (separation pulper) and/or a detrasher. Tub pulpers allow used paper diapers fed as starting materials to be efficiently crushed because they have a high disintegration ability as compared with drum pulpers.

Low-consistency pulpers include vertical continuous low-consistency tub pulpers from Maruishi Co., Ltd., and low-to-medium consistency pulpers from AIKAWA IRON WORKS CO., LTD., in which disintegration takes place at a consistency of about 3.0 to 8.0 %.

High-consistency pulpers include vertical batch high-consistency tub pulpers from AIKAWA IRON WORKS CO., LTD. and the like. Rotors that can be used in the pulpers include spiral rotors and HeliDisc rotors. Disintegration takes place at a consistency of around 8.0 to 20.0 %.

To remove contaminants such as plastics at early stages with high efficiency, a contaminant collector called rope ragger can be used in low-consistency and low-to-medium consistency pulpers.

Secondary pulpers include PAL sorters and PEA pulpers from AIKAWA IRON WORKS CO., LTD. and the like. These machines have the functions of mechanical disintegration and removal of coarse contaminants through a round hole strainer/basket or the like (coarse screening), thereby shortening the disintegration time in the primary pulper and promoting the separation of contaminants. Especially, PEA pulpers are suitable because they comprise a round hole strainer having a hole diameter of about 7.00 mm and a slot screen having a slot width of about 3.50 mm so that they remove contaminants with high efficiency.

Detrashers that can be used include screw separators and drum separators from AIKAWA IRON WORKS CO., LTD., and MAX drums combining a detrasher with a disintegration ability from AIKAWA IRON WORKS CO., LTD. can also be used as appropriate.

In the recycling process of paper diapers, the process time in the primary pulper can be shortened especially by using a secondary pulper because a lot of nonwoven fabrics are contained in the diapers and a lot of plastics are consumed for collecting them. Further, the efficiency of removing plastics and the like is more improved and workability is improved by employing a ragger even if a high-consistency pulper is used because the suspension of disintegrated diapers has high flowability.

Detrashers allow the volume of discharged residues to be reduced because they serve to dehydrate and finely screen starting materials discharged without being disintegrated in pulpers. Therefore, it is effective to treat used paper diapers in a detrasher after they have been disintegrated in a primary pulper and then treated in a secondary pulper.

For example, MAX drums from AIKAWA IRON WORKS CO., LTD. are effective for improving the recovery rate of fibers and SAPs and for reducing waste because they combine the dehydration/fine screening function of a detrasher and a disintegration ability so that starting materials discharged without being sufficiently disintegrated in pulpers can be treated and fibers and SAPs can be recovered therefrom, and therefore, MAX drums are more effectively used after pulpers to recover fibers.

In the present invention, disintegration takes place at a consistency of 3.0 to 20.0 % in pulpers because if the disintegration consistency is too low, SAPs tend to be readily swollen with an excessive amount of water and smaller amounts of paper diapers can be treated in a single run, while if the disintegration consistency is too high, the disintegration efficiency in pulpers decreases so that paper diapers cannot be sufficiently crushed. More preferably, the consistency is 3.0 to 15.0 %, even more preferably 3.0 to 8.0 %. After disintegration, the dispersion may be diluted to adjust the consistency to 0.3 to 2.0 %, preferably 0.3 to 1.5 %, more preferably 0.3 to 1.2 % in a tank, mixer or the like to prevent fibers from precipitating with SAPs.

<Screen>

Screens that can be used include inward flow or outward flow round hole and/or slot screens. Other screens that can be used include so-called reject screens or tail screens that are suitable for conditions involving high contaminant levels because they resist problems related to clogging or entanglement with contaminants, as well as composite screens combining a round hole screen with a basket-type slot screen (ADS double separator from AIKAWA IRON WORKS CO., LTD.).

The loss of fibers can be reduced and the contaminant separation efficiency can be increased by using a multistage screen system in which the reject from one screen is further treated by another screen. The multistage screen system may be a feed forward system in which the accepts from the secondary and subsequent screens are forwarded to the next steps or a cascade system in which they are returned to the previous stages, more preferably a feed forward system to improve the yield and to reduce the size of the system.

Further, a series (tandem) system comprising a round hole screen immediately followed by a slot screen may also be used. Round hole screens that can be used include those having a hole diameter of 3.00 to 0.50 mm, preferably 2.50 to 1.00 mm for coarse screening. If the hole diameter is greater than 3.00 mm, the contaminant removal efficiency decreases. If it is smaller than 0.5 mm, however, the runnability is impaired by possible clogging with gelled SAPs, because SAPs have a higher specific gravity than that of fibers and tend to be readily swollen into gels.

Slot screens that can be used include milled or bar-type baskets having a slot width of 0.30 to 0.10 mm, preferably 0.25 to 0.15 mm. If the slot width is greater than 0.30 mm, gelled SAPs are more likely to pass through the screen and hard to separate from fibers efficiently. If it is smaller than 0.10 mm, the runnability is impaired by possible clogging with gelled polymers.

Operating conditions of the screen in the present invention preferably include a solids content of 0.3 to 1.2 %, more preferably 0.4 to 0.8 %. Solids contents higher than 1.5 % are not preferred because the screen is liable to clogging, which results in a decrease in separation efficiency, while solids contents lower than 0.3 % are not preferred because a larger amount of liquid must be treated, which requires a longer process time and results in not only an increase in energy consumption but also an increase in post-screen dehydration load.

The screen flow through velocity is preferably in the range from 0.6 to 2.0 m/s, more preferably in the range from 1.0 to 1.5 m/s. Further, the peripheral velocity of the agitator in the screen is preferably 10 to 20 m/s, more preferably 14 to 18 m/s. Excessively low flow through velocities or peripheral velocities are not preferred, because gels are deposited on the basket or the like. However, excessively high flow through velocities or peripheral velocities are inconvenient, because the shear force excessively increases so that gelled SAP polymers are finely divided and hard to separate from fibers efficiently.

The recycling process of paper diapers is preferably a cascade process and/or feed forward process using two or more slot screens, more preferably a cascade process to improve the SAP removal efficiency.

<Cleaner>

Cleaners that can be used in the present invention include heavy contaminant cleaners using centrifugal force, preferably low-consistency/high-differential pressure or low-consistency/low-differential pressure cleaners.

In the present invention, the reject cone of the cleaner preferably has a diameter of 7.0 to 30.0 mm. Diameters smaller than 6.0 mm are not preferred because clogging of the reject cone makes it difficult to stably collect starting materials, while diameters greater than 30.0 mm are not preferred because the pressure difference between the inlet pressure and the outlet pressure of the reject cone is hard to control and the separation efficiency greatly decreases.

Operating conditions of the cleaner preferably include a solids content of 0.3 to 1.2 %, more preferably 0.3 to 0.8 %. Solids contents higher than 1.5 % are not preferred because the reject cone will be clogged so that starting materials cannot be stably collected, and even if they could be collected, they would not be sufficiently separated from contaminants, while solids contents lower than 0.3 % are not preferred because a larger amount of liquid must be treated, which requires a longer process time and results in not only an increase in energy consumption but also an increase in post-screen dehydration load.

The loss of fibers can be reduced and the contaminant separation efficiency can be increased by using a multistage process in which the reject from one cleaner is further treated by another cleaner. The multistage cleaner system may be a feed forward system in which the accepts from the secondary and subsequent cleaners are forwarded to the next steps or a cascade system in which they are returned to the previous stages, more preferably a feed forward system to improve the yield and to reduce the size of the system.

Further, a combination of a cleaner and a screen is also preferably used, more preferably a combination of two or more of a round hole screen and/or a slot screen, and a centrifugal cleaner, even more preferably a combination of a round hole screen, a centrifugal cleaner, and a slot screen in this order.

<Dehydrator/concentrator/washer>

In the present invention, dehydration/concentration/washing may take place in a system combining these functions or in separate units.

The medium-to-high consistency dehydrator is not specifically limited, so far as it is a machine by which a pulp having a consistency of about 2.0 to 3.0 % is dehydrated to about 10.0 % or a machine by which a pulp having a consistency of about 10.0 % is dehydrated to about 25.0 to 30.0 %, such as a screw thickener, an inclined extractor, a screw press or a power press.

The low-consistency concentrator is not specifically limited so far as it is a pre-dehydrator by which a pulp having a consistency of about 1.0 % is dehydrated/concentrated to a consistency of 3.0 % or more. For example, it may be a filtration/dehydration mechanism using a pulp mat such as a disc extractor or a disc thickener, or a spontaneous dehydration mechanism using a filtering/dehydrating element such as a SP filter or a trommel or the like.

Washers that can be used include high-speed washers/dehydrators such as wire and roll DNT washers, as well as horizontal drum-type trommels, fall washers, vertical washers Zekoo and the like.

Vertical washers are especially preferred for the recycling process of paper diapers because of low water consumption. As compared with horizontal dehydrators, the amount of washing water consumed can be saved to 1/3 or less. In addition, water consumption can be further saved by reusing the removed water as process water. For example, it can be used as diluting water at a stage preceding the screen/cleaner.

<High-consistency processor>

The high-consistency process in the present invention takes place at a consistency of 10.0 to 50.0 %, preferably 15.0 to 45.0 %, more preferably 20.0 to 40.0 %. High-consistency processors include low-speed kneaders, high-speed dispersers and the like. In Flow 2 shown above, a hot dispersion system of high ability to disperse contaminants is preferably used as the high-consistency processor. In Flow 3, a kneader is preferably used as the high-consistency processor because SAPs are thermally modified into resin particles which are easy to separate, more preferably followed by screening.

Kneaders that can be used include single-screw, twin-screw and four-screw kneaders as well as kneaders having two or more kneading sections. SAPs can be thermally modified into resin particles by releasing water contained therein by applying steam to the kneading sections to heat them or by continuously passing the material through the multiple kneading sections to bring about spontaneous heat generation by friction between fibers, thereby raising the process temperature to 40 to 120 °C, preferably 40 to 100 °C, more preferably 50 to 80 °C. It should be noted that in the present invention, heating can also take place at stages other than the high-consistency process to improve the efficiency of separating fibers and SAPs. Heating seems to promote water release from SAPs because the bonding force between carboxyl groups of polyacrylic acid in SAPs and water molecules decreases. Further, heating is also preferred because soiled water contained in SAPs can be readily replaced/disinfected. In addition, the drying efficiency after recovery of SAPs can also be expected to be improved because the moisture content of SAPs decreases.

Dispersers that can be used include disc dispersers and conical dispersers. So-called hot dispersion can take place to finely disperse SAPs and make them invisible by combining a disperser with a heating tube or the like located immediately before it.

3. Chemical additives and the like

The present invention relates to the processes wherein the step of disintegrating used paper diapers and dispersing them in water comprises adding a bleaching/microbicidal agent, a crosslinking agent and an acidic agent to treat them.

<Bleaching/microbicidal agent>

A bleaching/microbicidal agent is added for the purpose of subjecting soils contained in paper diapers fed as starting materials and soiled components such as fibers and SAPs to a bleaching and disinfecting/microbicidal treatment. The bleaching/microbicidal agent is preferably added to a machine that disintegrates used paper diapers such as a pulper. This is an efficient method because starting materials containing soiled water are together subjected to a strong bleaching/microbicidal treatment in advance.

Bleaching/microbicidal agents are classified into two types, i.e., oxidizing and reducing agents, and oxidizing agents are further classified into chlorine-based and oxygen-based agents. Among them, chlorine-based oxidizing agents are preferred because of their high oxidizing power, especially sodium hypochlorite because it has the highest oxidizing power and allows an efficient bleaching/microbicidal treatment.

In the present invention using starting materials containing human feces, it is efficient to add sodium hypochlorite because it has a high microbicidal/bleaching effect in the weakly acidic to neutral pH range within which the process pH seems to fall. On the other hand, patent document 1 cited above describes using hydrogen peroxide (oxygen-based oxidizing bleach) and a microbicide (peracetic acid), but their bleaching or microbicidal effect is low as compared with sodium hypochlorite. To attain an optimal bleaching/microbicidal effect in patent document 1, the pH must be controlled in the alkaline range because hydrogen peroxide and the microbicide are more effective at pH 11.0 or more and pH 9.0 or more respectively, but if the pH is high (pH 11.0 or more), the sodium ion concentration increases and the effect of the crosslinking agent decreases, and in addition, fibers undergo alkali yellowing, which may impede improvements in brightness. If the pH is acidic (pH 4.0 or less), however, fibers are damaged (i.e., the degree of polymerization of cellulose decreases) so that the strength of the fibers decreases, which may cause problems when they are reused. In the present invention, the treatment can be achieved without the necessity of pH control (alkali treatment) nor any adverse effect on fibers by using sodium hypochlorite that has a high microbicidal/bleaching effect in the neutral to weakly acidic pH range.

The proportion of the bleaching/microbicidal agent to be added is 1 to 100000 ppm, preferably 5 to 30000 ppm, more preferably 10 to 20000 ppm in the dispersion at the instant when it is added. If it is 1 ppm or less, there is a possibility that a sufficient bleaching/microbicidal effect cannot be expected, but if it is 100000 ppm or more, much sodium sulfite is needed to reduce free chlorine at the subsequent stage, which will be uneconomical, as described later.

<Crosslinking agent>

A crosslinking agent is added to crosslink carboxyl groups of polyacrylic acid constituting a main component of SAPs and thus to inhibit swelling of SAPs. The crosslinking agent is effectively added to a machine that disintegrates used paper diapers such as a pulper in the same manner as the bleaching/microbicidal agent.

The crosslinking agent may be any polyvalent metal salt including, for example, calcium chloride, calcium nitrate, calcium sulfate, calcium hydroxide, calcium carbonate, magnesium chloride, magnesium nitrate, magnesium sulfate, magnesium hydroxide, magnesium carbonate, aluminum sulfate, aluminum polychloride (PAC) and the like. Among them, calcium chloride is preferred because it is inexpensive and highly effective for inhibiting SAPs from swelling.

The proportion to be added may be 1.0 to 30.0 %, preferably 5.0 to 20.0 %, more preferably 10.0 to 20.0 % based on the weight of the starting material. If it is 1.0 % or less, the crosslinking agent cannot be sufficiently expected to produce its effect, but if it is 30.0 % or more, the advantages of the anti-swelling effect of crosslinking on SAPs are outweighed by the disadvantages of an increased risk of corrosion in process piping by excessive chloride ions.

How and when the crosslinking agent is to be added are not specifically limited, and it may be added when sanitary products are dispersed in an aqueous medium to prepare a slurry. The crosslinking agent may be added at a time, or in portions, or continuously.

<Acidic agent>

An acidic agent is added to lower the pH and allow the dispersion to turn into an acidic solution, thereby improving the efficiency of separating fibers and SAPs. When the pH decrease, the separation/recovery efficiency can be improved because SAPs form sand-like fine resin particles rather than a gel so that the screen is no more clogged with them. This may be explained as follows: Polyacrylic acid constituting a main component of SAPs has an acid dissociation constant pKa of about 5.3 to 5.7, and therefore, it shifts from the ionic state to the free acid state at a pH in this range or below so that water contained in SAPs is released.

The acidic agent can be added after adding the crosslinking agent, and effectively added to a pulper or a chest after dilution. To prevent fibers from precipitating with SAPs, the consistency may be adjusted to 0.3 to 2.0 %, preferably 0.3 to 1.5 %, more preferably 0.3 to 1.2 %.

The acid that can be used as the acidic agent is not specifically limited, and may be either an organic acid or an inorganic acid (mineral acid), but inorganic acids are preferred because they are inexpensive and small amounts are required to lower the pH, among which sulfuric acid and aluminum sulfate are especially preferred.

The amount to be added may be controlled to adjust the dispersion at pH 6.0 or less after it has been added, preferably pH 4.0 to 6.0, more preferably pH 4.5 to 5.5, even more preferably pH 4.5 to 5.3.

When aluminum sulfate is used to adjust the pH, it must be added in higher amounts because it has a small effect on pH decrease for the amount added. As a result, the sulfate ion concentration in the system increases so that calcium sulfate (gypsum) scale may be formed with calcium ions of calcium chloride or the like added as the crosslinking agent. To prevent this, sulfuric acid is effectively used because it has a greater effect on pH decrease with smaller amounts added.

<Reducing agent>

A reducing agent may be added to reduce free chlorine in the system. When the pH is lowered with the acidic agent while free chlorine (residual chlorine) of sodium hypochlorite added in excess as the bleaching/microbicidal agent remains, chlorine gas is generated. To avoid this, the acidic agent is desirably added after free chlorine has been reduced in advance. Sodium sulfite can be preferably used as the reducing agent. Whether or not the reducing agent should be added and the amount of the reducing agent to be added can be determined by measuring free chlorine in the supernatant of the dispersion using a kit such as Pack Test during the step where the reducing agent is used. Typically, the reducing agent can be added in an amount of 0.0 to 50.0 % of sodium hypochlorite added, but the present invention is not specifically limited to these values.

Processes for treating sanitary products

We describe processes for treating sanitary products containing a fiber and a SAP, whereby the fiber and the SAP can be efficiently separated and recovered from the sanitary products.

As already described, the present invention comprises the step of disintegrating a used sanitary product and dispersing it in water to give a slurry. In this step, a crosslinking agent for crosslinking the SAP, a bleaching/microbicidal agent and an acidic agent for facilitating the separation of the SAP are added. In preferred embodiments, a reducing agent may be added to the slurry. This step is preferably performed using a pulper preferably in combination with a detrasher.

Further, the present invention comprises the step of separating and recovering the fiber and SAP contained in the sanitary product. This step is preferably performed using a screen or a cleaner, whereby the fiber and SAP can be efficiently obtained from the slurry.

Further in the present invention, the slurry containing the fiber may be dehydrated and the water thus obtained may be reused.

According to the present invention, fibers and SAPs can be obtained from used sanitary products, which otherwise would have been disposed of as waste, because the fibers and SAPs can be efficiently separated and recovered from the sanitary products.

EXAMPLES

The following examples further illustrate the present invention by taking paper diapers as examples of sanitary products, but the present invention is not limited to the examples below. Unless otherwise specified, the parts and % as used herein refer to parts by weight and % by weight, respectively, and the numerical ranges are intended to include their endpoints.

Various evaluation methods

(1) Methods for determining fiber properties

(Method for determining freeness CSF) It was measured according to "the Determination of drainability - Canadian standard freeness method" defined in JIS P 8121:1995.

(Methods for determining average fiber length, fiber width, curl index and fines content) Length weighted average fiber length and fiber width were measured using Fiber Tester (from Lorentzen & Wettre). Further, the content of fibers having a fiber length of 0.2 mm or less was reported as fines content. Furthermore, "100 - <shape factor>" was reported as the curl index of fibers.

(Methods for determining freeness CSF after disintegration and water retention value) Ten handsheets having a bone dry basis weight of 60 g/m$^2$ prepared according to JIS P 8209 were disintegrated according to JIS P 8220:1998 to prepare test samples. These samples were measured for their Canadian standard freeness (CSF) according to JIS P 8121:1995, and measured for their water retention value expressed as a percentage based on the bone dry weight according to JAPAN TAPPI No 26:2000.

(2) Methods for quantifying dirt levels

(Composition analysis of starting materials by an enzymatic assay) To 300 ml (2 g on a bone dry weight basis) of a slurry of each starting material having a solids content of 0.7 % adjusted to pH 4.5 was added 2 % by weight of an enzyme (Acremonium from Meiji Seika Kaisha, Limited) and the mixture was reacted at 50 °C for 24 hours to degrade pulp fibers formed of cellulose into monosaccharides. On the premise that this solution mainly contains SAPs, nonwoven fabrics and plastics and when it is stirred, the SAPs will settle while the nonwoven fabrics and the plastics will remain in the supernatant, the SAPs were separated from the nonwoven fabrics and the plastics by repeating the cycle of stirring the solution and discarding the supernatant until the supernatant no more contained nonwoven fabrics and plastics and became clear. Then, the weight of pulp was calculated from the weights of both fractions to analyze the composition of the starting material, whereby the SAP content was determined.

(Method for quantifying SAP levels by a staining assay) To 300 ml (2 g on a bone dry weight basis) of a slurry of each starting material having a solids content of 0.7 % was added a 4 % aqueous cobalt (II) chloride hexahydrate solution to stain SAP particles, and then handsheets having a basis weight of 60 g/m$^2$ were prepared using a cylinder handsheet machine according to JIS P 8209, and measured for the number and the area of SAP particles stained in dark blue per 1 square meter using the dirt analyzer EasyScan (from Nippon Paper Unitec Co., Ltd.) according to the measurement conditions of JIS P 8208.

(Dirt assay) The same procedure as described for the method for quantifying SAP levels was followed except that SAP particles were not stained with an aqueous cobalt (II) chloride hexahydrate solution. It should be noted that unstained and dried SAP particles are not counted as dirt particles because they are white.

(3) Analyses of strength properties of handsheets

Handsheets having a bone dry basis weight of 60 g/m$^2$ were prepared according to JIS P 8209 and analyzed for the following properties.

(Density) Calculated from the measured values of the paper thickness and the basis weight of the handsheets. The paper thickness was measured according to JIS P 8118:1998, and the basis weight was measured according to JIS P 8124:1998.

(Brightness) Measured according to JIS P 8148:2001.

(Opacity) Measured according to JIS P 8149:2000.

(Breaking length) Measured according to JIS P 8113:1998.

(Young's modulus) Calculated from the values measured using L&W TENSILE TESTER (from Lorentzen & Wettre).

(Bending stiffness) The bending stiffness was measured at a bending angle of 15 ° according to ISO-2493 using L&W Bending Tester Cord 160 (from Lorentzen & Wettre).

(Burst index) Measured according to JIS P 8131:2009.

(Tear index) Measured according to JIS P 8116:2000.

(Rate of change in hygroscopicity) The handsheets were humidified under conditions of humidity 80 %, temperature 23 °C for 12 hours and weighed. Immediately after then, they were dried in a dryer at 105 °C for 3 hours and measured for their dry weight.

$$\text{Rate of change in hygroscopicity (\%)} = (\text{Weight at humidity 80 \% - Dry weight}) / (\text{Dry weight}) \times 100.$$

(Knot content) Fluff pulp (0.2 g on a bone dry basis) was collected and spread on water filled in a vat, and the number of pulp fiber bundles was counted and reported as knot content (counts/g).

(Bulkiness) Fluff pulp (4 g on a bone dry basis) was collected and filled in a clear plastic tube of 54 mm in diameter having a 40-mesh wire screen at the bottom and loaded with a metallic weight of 580 g placed on the top, and the resulting fluff pulp structure was measured for the height (mm).

(Water absorption speed) Tested according to the method described in JPA 2009-148441. The time (seconds) taken for 40 ml of 0.9 % physiological saline to be completely absorbed was determined using a dedicated measuring device.

(Total volume of water absorbed) Tested according to the method described in JPA 2009-148441. First, a thin absorbent article pre-measured for the dry weight was immersed in a sufficient amount of 0.9 % physiological saline and left for 5 minutes. Then, the thin absorbent article was lifted and drained with the top sheet 2 facing downward on a wire screen for 30 seconds and then measured for the weight to determine the total volume absorbed from the weights before and after absorption (assuming that the difference between the weights before and after absorption of 1 g equals the volume of 1 ml absorbed).

(Wet-back) Into the center of an absorbent article was injected 250 ml of 0.9 % physiological saline, and after a lapse of 10 minutes, a pre-weighed filter paper (No. 2 filter paper having a diameter of 55 mm from ADVANTEC) was put on the center of the injection site, and a weight of 687 g was placed on the filter paper (pressure: 35 g/cm$^2$). One minute after the weight was placed, the weight of the filter paper was measured, and the difference (g) between the weights of the filter paper before and after testing was determined and reported as wet-back.

(Appearance evaluation) The handsheets were heated in a dryer at 180 °C for 30 minutes, and evaluated for their surface appearance by touch and visual inspection on the 3-class scale below:
○: No change from before drying; ∆: Some irregularities are observed on the surface as compared with before drying;

x: Marked irregularities occur by drying, which not only impair appearance but also cause strength problems in construction materials or the like.

Experiment 1: Evaluation test of separation of SAPs (1)

(Comparative example 1-1: Starting material A)

In a high-consistency pulper (from AIKAWA IRON WORKS CO., LTD.), 400 kg of fiber sheets recycled from used paper diapers (commercially available as construction materials obtained by using calcium chloride for treating SAPs) were crushed to a solids content of 8 % under conditions of a temperature of 40 °C for 10 minutes and then diluted to a solids content of 0.7 % to collect a sample.

(Comparative example 1-2)

NBKP dry sheets (NBKP: unbleached kraft pulp from Rayonier) typically used for paper diapers were disintegrated according to JIS P 8220:1998 to collect a sample.

(Example 1-1: Starting material B)

Starting material A was adjusted to pH 5.1 with sulfuric acid, and then treated using the Lamor cleaner model LCC-150 having a reject nozzle diameter of 8 mm (from AIKAWA IRON WORKS CO., LTD.) under conditions of an inlet piping pressure of 0.320 MPa, a flow rate of 0.54 $m^3$/min, and an outlet piping pressure of 0.065 MPa.
Then, the resulting material was treated using the screen model GFC-400 having a slot width of 0.15 mm (from AIKAWA IRON WORKS CO., LTD.) under conditions of an inlet piping pressure of 0.110 MPa, a flow rate of 0.70 $m^3$/min, an outlet piping pressure of 0.060 MPa, and a screen flow through velocity of 1.0 m/sec.
Further, the resulting material was washed using a 60-mesh trommel type A (from AIKAWA IRON WORKS CO., LTD.), and dehydrated to a solids content of about 30 % using an inclined extractor and a screw press (both from AIKAWA IRON WORKS CO., LTD.) to collect a sample.

(Example 1-2)

Starting material A was adjusted to pH 5.1 with sulfuric acid, and then treated using the screen model GFC-400 having a slot width of 0.15 mm under conditions of an inlet piping pressure of 0.110 MPa, a flow rate of 0.70 $m^3$/min, an outlet piping pressure of 0.060 MPa, and a screen flow through velocity of 1.0 m/sec to collect a sample.

(Example 1-3) (Comparative Example, for reference)

Starting material A was adjusted to pH 5.1 with sulfuric acid, and then treated using the screen model GFC-400 having a slot width of 0.20 mm under conditions of an inlet piping pressure of 0.110 MPa, a flow rate of 0.70 $m^3$/min, an outlet piping pressure of 0.060 MPa, and a screen flow through velocity of 1.0 m/sec to collect a sample.

<Evaluation results>

The evaluation results of the samples described above are shown in Table 1
The composition of each starting material was analyzed by an enzymatic assay. In contrast to Comparative example 1-1 containing 40 % or more of components other than pulp fibers including 13.5 % of SAPs and 28.1 % of nonwoven fabrics or the like, Example 1-1 comprises 91.6 % of pulp and as little as 0.3 % of SAPs which may have adverse influence especially on appearance and strength. The SAP content of Example 1-2 was 2.3 %, and the SAP content of Example 1-3 was 6.1 %.
Similarly, the SAP levels of Example 1-1 were also very low as compared with Comparative example 1-1 when determined by a staining assay. Dirt levels other than SAPs in Example 1-1 also decreased to 1/5 or less of those observed in Comparative example 1-1. The SAP levels of Example 1-2 and Example 1-3 determined by the staining assay and the enzymatic assay also tended to show similar results. Further, dirt levels other than SAPs in Example 1-2 and Example 1-3 also decreased to 1/3 or less and about 1/2 respectively as compared with Comparative example 1-1.
On the other hand, no changes were observed in freeness and fiber length, indicating that the present recycling process does not have any adverse influence on pulp fiber properties and causes no problem in strength. The strength such as breaking length deceased with the increase in the SAP content of recycled fibers, while the optical properties such as brightness and opacity were improved with the increase in the proportion of pulp in recycled fibers. In Example 1-1, recycled fibers having optical properties and strength comparable to those of Comparative example 1-2 (NBKP) were successfully obtained.

Further, the Examples of the present invention had a high water retention value and a high rate of change in hygroscopicity as compared with the Comparative examples containing much SAPs, showing that they are more likely to contain water when they are used as starting materials. As for the strength of handsheets, the Examples of the present invention showed no changes in appearance and retained high size stability in contrast to the Comparative examples that showed marked irregularities.

Especially, the Examples of the present invention were comparable to NBKP (Comparative example 1-2) in strength and appearance. The recycled fibers according to the present invention seem to be of sufficient quality to serve as fibers used for reinforcing construction materials which are, in general, often prepared by autoclaving and incubation at high temperatures (for example, about 180 °C). Further, the recycled fibers of the present invention seem to be also well-suitable for use in cushioning materials, molded products and the like as alternative to NBKP.

[Table 1]

| Sample | | Fiber properties | | | | | |
|---|---|---|---|---|---|---|---|
| | | Freeness after disintegration CSF (ml) | Fiber length (mm) | Fiber width (μm) | Curl index (%) | Fines content (%) | Water retention value (%) |
| Comparative example 1-1 | Commercially available recycled fiber | 746 | 2.20 | 32.3 | 13.2 | 2.5 | 140 |
| Comparative example 1-2 | Commercially available NBKP | 733 | 2.73 | 34.6 | 11.9 | 4.5 | 121 |
| Example 1-1 | Screening + Cleaning | 724 | 2.23 | 31.1 | 11.3 | 2.2 | 125 |
| Example 1-2 | Screening | 733 | 2.28 | 32.1 | 12.5 | 2.3 | 122 |
| Comparative example 1-3 | Screening | 739 | 2.24 | 31.8 | 13.5 | 2.5 | 121 |

| Sample | | Dirt levels | | | | | | |
|---|---|---|---|---|---|---|---|---|
| | | Enzymatic assay Weight (%) | | | Staining assay (60 g/m2 handsheet) | | Dirt assay (60 g/m2 handsheet) | |
| | | Pulp | SAP | Nonwovens, plastics | Number of particles Counts/m2 | Area mm2/m2 | Number of particles Counts/m2 | Area mm2/m2 |
| Comparative example 1-1 | Commercially available recycled fiber | 58.4 | 13.5 | 28.1 | 160,000 | 68,000 | 2,100 | 291 |
| Comparative example 1-2 | Commercially available NBKP | 100.0 | - | - | 100 | 5 | 33 | 5 |
| Example 1-1 | Screening + Cleaning | 91.6 | 0.3 | 8.1 | 4,400 | 440 | 340 | 37 |
| Example 1-2 | Screening | 89.3 | 2.3 | 8.4 | 30,000 | 5,400 | 600 | 68 |
| Comparative example 1-3 | Screening | 85.9 | 6.1 | 8.0 | 90,000 | 33,000 | 1,100 | 180 |

| | Sample | Handsheet | | | | | | | | | | |
|---|---|---|---|---|---|---|---|---|---|---|---|---|
| | | Basis weight | Density | Brightness | Opacity | Breaking length | Young's modulus | Bending stiffness | Burst index | Tear index | Rate of change in hygroscopicity | Appearance evaluation |
| | | g/m2 | g/m3 | % | % | km | GPa | μN.m | kPa/g/m2 | mN/g/m2 | % | |
| Comparative example 1-1 | Commercially available recycled fiber | 68.5 | 0.47 | 80.9 | 70.1 | 1.8 | 1.1 | 136 | 1.3 | 11.6 | 14.2 | × |
| Comparative example 1-2 | Commercially available NBKP | 67.1 | 0.44 | 84.4 | 72.8 | 2.4 | 1.9 | 251 | 1.6 | 14.9 | 11.9 | ○ |
| Example 1-1 | Screening + Cleaning | 66.0 | 0.48 | 83.4 | 71.3 | 2.4 | 1.9 | 172 | 1.6 | 16.0 | 11.6 | ○ |
| Example 1-2 | Screening | 65.9 | 0.47 | 81.9 | 71.9 | 2.2 | 1.5 | 169 | 1.5 | 13.8 | 11.8 | △ |
| Comparative example 1-3 | Screening | 66.0 | 0.47 | 80.9 | 69.7 | 2.1 | 1.3 | 154 | 1.4 | 11.4 | 11.4 | △ |

Experiment 2: Evaluation test of separation of SAPs (2)

(Reference example 1: Starting material C)

Two kilograms of unused paper diaper pants and pads from NIPPON PAPER CRECIA Co., LTD. were impregnated with 4.6 kg of physiological saline (a 0.9 % sodium chloride solution) to mimic urine, and packed into plastic garbage bags for burnable waste.

One hundred and ten such plastic bags, which amount to 220 kg of paper diaper waste, were thrown into a high-consistency pulper (from AIKAWA IRON WORKS CO., LTD.) filled with a 10 % calcium chloride solution at a water temperature of 40 °C to reach a solids content of 8 %, and adjusted to pH 4.7 with concentrated sulfuric acid, then crushed for 10 minutes, diluted to a solids content of 0.7 %, and then treated using a 2.2 mm round hole screen (model FR-400 from AIKAWA IRON WORKS CO., LTD.) under conditions of an inlet piping pressure of 0.10 MPa, a flow rate of 1.01 $m^3$/min, and an outlet piping pressure of 0.05 MPa to collect a sample (starting material C).

(Comparative example 2-1: Starting material D)

Starting material C was washed in a trommel type A in the same manner as in Example 1-1 to collect a sample (starting material D). Specifically, the starting material was washed using a 60-mesh trommel type A (from AIKAWA IRON WORKS CO., LTD.), and dehydrated to a solids content of about 30 % using an inclined extractor and a screw press (both from AIKAWA IRON WORKS CO., LTD.) to collect a sample.

(Example 2-1: Starting material E)

Starting material C was treated using the screen model GFC-400 having a slot width of 0.20 mm under the same conditions as in Example 1-1 to collect a sample (starting material E). Specifically, the starting material was treated using the screen model GFC-400 having a slot width of 0.15 mm (from AIKAWA IRON WORKS CO., LTD.) under conditions of an inlet piping pressure of 0.110 MPa, a flow rate of 0.70 $m^3$/min, an outlet piping pressure of 0.060 MPa, and a screen flow through velocity of 1.0 m/sec.

(Example 2-2: Starting material F)

Starting material C was treated using the screen model Max 0-400 having a slot width of 0.20 mm (from AIKAWA IRON WORKS CO., LTD.) under conditions of an inlet piping pressure of 0.09 MPa, an accept side piping pressure of 0.06 MPa, and a screen flow through velocity of 1.2 m/sec to collect a sample (starting material F).

(Example 2-3: Starting material G)

Starting material C was treated using the Cyclotec cleaner model CT-15 having a reject nozzle diameter of 30 mm (from AIKAWA IRON WORKS CO., LTD.) under conditions of an inlet piping pressure of 0.180 MPa, a flow rate of 1.20 $m^3$/min, and an outlet piping pressure of 0.030 MPa to collect a sample (starting material G).

(Example 2-4: Starting material H)

Starting material C was treated using the Lamor cleaner model LCC-150 under the same conditions as in Example 1-1 to collect a sample (starting material H). Specifically, the starting material was treated using the Lamor cleaner model LCC-150 having a reject nozzle diameter of 8 mm (from AIKAWA IRON WORKS CO., LTD.) under conditions of an inlet piping pressure of 0.320 MPa, a flow rate of 0.54 $m^3$/min, and an outlet piping pressure of 0.065 MPa.

(Example 2-5: Starting material I)

Starting material C was treated using the screen model Max 0-400 under the same conditions as for starting material F, and subsequently treated using the Lamor cleaner model LCC-150 under the same conditions as for starting material H to collect a sample (starting material I).

<Evaluation results>

The measurement results of dirt levels in the samples described above are shown in Table 2.

Comparative example 2-1 (starting material D) prepared by washing starting material C obtained by impregnating unused sanitary products with physiological saline and then disintegrating them in a washer had a high area fraction when determined by a staining assay, which means that it has high SAP levels. Further, it was poor in appearance and showed marked irregularities sensed by touch. In Example 2-1 (starting material E) and Example 2-2 (starting material F) obtained by screening, the SAP area was too small to sense by touch especially because particles much greater than the slot width of the screen have been removed. In Example 2-3 (starting material G) and Example 2-4 (starting material H) obtained by cleaning, the SAP area was tiny in the recovered samples of recycled fibers because 99 % or more of SAPs have been eliminated as heavy contaminants outside the system.

In Example 2-5 (starting material I) obtained by screening followed by cleaning, SAP removal in the cleaner seems to be more effective because cleaning takes place after nonwoven fabrics and plastics have been removed by a screen so that clogging with nonwoven fabrics and plastics during cleaning is reduced.

[Table 2]

|  | Sample | Dirt levels | | | |
|---|---|---|---|---|---|
|  |  | Staining assay | | Dirt assay | |
|  |  | Counts/m2 | mm2/m2 | Counts/m2 | mm2/m2 |
| Comparative example 2-1 | Starting material D | 160,000 | 92,000 | 2,000 | 262 |
| Example 2-1 | Starting material E | 150,000 | 28,000 | 620 | 72 |
| Example 2-2 | Starting material F | 170,000 | 38,000 | 1,200 | 165 |
| Example 2-3 | Starting material G | 2,100 | 340 | 350 | 46 |
| Example 2-4 | Starting material H | 1,200 | 200 | 500 | 79 |
| Example 2-5 | Starting material I | 380 | 36 | 140 | 15 |
| Reference example 1 | Starting material C | 260,000 | 140,000 | 2,700 | 370 |

Experiment 3: Preparation of fluff pulps

<Preparation of roll sheets>

Example 1-1 (a recycled fiber derived from sanitary products) and Comparative example 1-2 (NBKP) obtained in Experiment 1 were used in various mixing ratios to prepare three roll sheets having a basis weight of 300 g/m$^2$ and a density of 0.44 to 0.47g/cm$^3$ in a test machine from Awa Paper Mfg. Co., Ltd.

(Comparative example a) A roll sheet containing 100 % of NBKP.

(Example a) A roll sheet prepared from a mixture of NBKP and the recycled fiber derived from sanitary products in a ratio of 8:2.

(Example b) A roll sheet prepared from a mixture of NBKP and the recycled fiber derived from sanitary products in a ratio of 6:4.

(Example c) A roll sheet containing 100 % of the recycled fiber derived from sanitary products.

<Evaluation results>

The roll sheets described above were disintegrated, and analyzed for their freeness, water retention value, and fiber properties. Further, handsheets having a basis weight of 60 g/m$^2$ were prepared and analyzed for their brightness, dirt area, and burst index. The results are shown in Table 3.

In any of the Examples incorporating 20 to 100 % of the recycled fiber of the present invention, no significant difference was observed in freeness and water retention value as compared with Comparative example a using NBKP (virgin pulp) known to be used as fluff pulp for paper diapers or the like.

As the proportion of the recycled fiber increased, fiber properties showed the following tendency: the fiber length and the fiber width decreased, the curl index increased, and the fines content decreased. This may be contributed to the recovery process of the recycled fiber as well as the difference of the starting materials used.

On the other hand, the brightness and the burst index were comparable to those of NBKP alone (Comparative example a) at any proportion of the recycled fiber. The dirt area indicative of the dirt levels of the sample containing 100 % of the recycled fiber (Example c) was also sufficiently small, i.e., 40 mm$^2$/m$^2$ or less.

It was concluded from these results that the roll sheets prepared from the recycled fiber obtained here can be sufficiently used for fluff pulp applications.

[Table 3]

| | Recycled fiber content | Freeness after disintegration | Water retention value | Fiber length | Fiber width | Curl index | Fines content | Brightness | Dirt area | Burst index |
|---|---|---|---|---|---|---|---|---|---|---|
| | % | ml | % | mm | μm | % | % | % | mm2/m2 | kPa/g/m2 |
| Comparative example a | 0 | 701 | 121 | 2.73 | 34.6 | 11.9 | 4.5 | 84.4 | 5.2 | 1.55 |
| Example a | 20 | 720 | 122 | 2.52 | 32.8 | 12.3 | 3.9 | 85.9 | 3.6 | 1.43 |
| Example b | 40 | 685 | 126 | 2.49 | 32.5 | 12.7 | 3.3 | 85.3 | 10.5 | 1.51 |
| Example c | 100 | 699 | 127 | 2.25 | 31.5 | 13.1 | 2.2 | 84.2 | 38.7 | 1.50 |

<Preparation of fluff pulps>

The roll sheets described above were stacked to a basis weight of 600 to 1050 g/m$^2$ and disintegrated using a disintegrator having a toothed cylinder (from ZUIKO CO., LTD.) at a rotation speed of 3600 rpm, a sheet feeding velocity of 15 m/min, and a clearance of 0.8 mm to prepare fluff pulps.

(Comparative example 3-1) A fluff pulp containing 0 % of the recycled fiber (100 % of NBKP).
(Example 3-1) A fluff pulp containing 6 % of the recycled fiber.
(Example 3-2) A fluff pulp containing 11 % of the recycled fiber.
(Example 3-3) A fluff pulp containing 29 % of the recycled fiber.
(Example 3-4) A fluff pulp containing 30 % of the recycled fiber.
(Example 3-5) A fluff pulp containing 70 % of the recycled fiber.

<Evaluation results>

The fluff pulps described above were analyzed for their fiber properties, knot content and bulkiness. The results are shown in Table 4.
The fiber properties of the fluff pulps containing 6 to 70 % of the recycled fiber were not significantly different from the fiber properties of the fluff pulp prepared solely from NBKP (Comparative example 3-1). Further, no difference was observed in the measurement results of the knot content and bulkiness between the fluff pulps prepared with the recycled fiber and the fluff pulp prepared from 100 % of NBKP.
As described above, the fluff pulps prepared from the the recycled fiber according to the present invention had performances comparable to those of the fluff pulp prepared from 100 % of NBKP.

[Table 4]

| | Recycled fiber content | Fiber length | Fiber width | Curl index | Fines content | Knot content | Bulkiness |
|---|---|---|---|---|---|---|---|
| | % | mm | $\mu$m | % | % | counts/g | mm |
| Comparative example 3-1 | 0 | 2.55 | 33.7 | 14.9 | 1.0 | 175 | 24.6 |
| Example 3-1 | 6 | 2.22 | 33.3 | 14.3 | 1.9 | 400 | 24.2 |
| Example 3-2 | 11 | 2.31 | 32.9 | 14.8 | 1.2 | 450 | 24.3 |
| Example 3-3 | 29 | 2.41 | 33.2 | 14.7 | 1.5 | 205 | 24.8 |
| Example 3-4 | 30 | 2.49 | 32.4 | 14.0 | 1.1 | 340 | 24.1 |
| Example 3-5 | 70 | 2.34 | 31.7 | 15.0 | 1.0 | 160 | 24.5 |

Experiment 4: Preparation of incontinence pads

Incontinence pads were prepared according to the method described in JPA 2009-148441. They comprise a top sheet consisting of an air through bonded nonwoven fabric (basis weight 25 g/m$^2$); a back sheet consisting of a cloth-like back sheet (basis weight 35 g/m$^2$) made of a polyethylene film laminated with a nonwoven fabric on the outside; a transfer sheet consisting of an air through bonded nonwoven fabric (30 g/m$^2$); and a water-repellent sheet and a side sheet both consisting of a spunbond nonwoven fabric (20 g/m$^2$). Further, the SAP content of the absorbent core was 40 % by mass. The fluff pulps obtained in Experiment 3 were used.
(Comparative example 4-1) An incontinence pad prepared with the fluff pulp of Comparative example 3-1.

(Example 4-1) An incontinence pad prepared with the fluff pulp of Example 3-1.

(Example 4-2) An incontinence pad prepared with the fluff pulp of Example 3-2.

(Example 4-3) An incontinence pad prepared with the fluff pulp of Example 3-3.

(Example 4-4) An incontinence pad prepared with the fluff pulp of Example 3-4.

(Example 4-5) An incontinence pad prepared with the fluff pulp of Example 3-5.

<Evaluation results>

The incontinence pads prepared as described above were evaluated for their water absorption speed, total volume absorbed, and wet-back. The results are shown in Table 5.

All of the samples incorporating 6 to 70 % of the recycled fiber had sufficient performances for practical uses as proved by the fact that their water absorbing properties of the incontinence pads such as water absorption speed and the volume absorbed were approximately comparable to those of the sample prepared from 100 % of NBKP. Further, all of them were aesthetically pleasing with no difference in appearance fault or the like from the sample prepared from of 100 % of NBKP.

Therefore, the incontinence pads using the recycled fiber of the present invention successfully achieved quality levels comparable to those of the incontinence pad prepared from of 100 % of NBKP.

[Table 5]

|  |  | Comparative example 4-1 | Example 4-1 | Example 4-2 | Example 4-3 | Example 4-4 | Example 4-5 |
|---|---|---|---|---|---|---|---|
| Recycled fiber content | % | 0 | 6 | 11 | 29 | 30 | 70 |
| Water absorption speed | sec/ 40ml | 51.9 | 47.8 | 51.8 | 53.0 | 54.9 | 60.8 |
| Total volume absorbed | ml | 685 | 668 | 675 | 668 | 662 | 657 |
| Wet-back | g | 0.4 | 0.2 | 0.3 | 0.4 | 0.3 | 0.3 |

Experiment 5: Strength test of the recycled pulps

The samples used in Experiment 1 and Experiment 3 were evaluated for their fiber properties, and the dirt levels and strength of the handsheets. The evaluation results are shown in Table 6.

The Examples of the present invention were superior to Comparative example 1-1 containing much SAPs and nearly comparable to NBKP (Comparative example 1-2) in strength properties such as breaking length.

Further, both of the number and the area of dirt particles in the Examples of the present invention were smaller than those of Comparative example 1-1 containing much SAPs and nearly comparable to those of NBKP (Comparative example 1-2). Given that pulps especially used for writing and printing papers are required to have a low dirt content, the recycled fibers of the present invention can be used as alternative to NBKP as starting materials for writing and printing papers such as newsprint paper and PPC paper; and tissue papers (sanitary papers) such as facial tissue and paper towels because they also have high brightness.

[Table 6]

| | Fiber properties | | | | | |
|---|---|---|---|---|---|---|
| | Freeness after disintegration CSF | Fiber length | Fiber width | Curl index | Fines content | Water retention value |
| | ml | mm | μm | % | % | % |
| Comparative example 1-1 | 746 | 2.20 | 32.3 | 13.2 | 2.5 | 140 |
| Comparative example 1-2 | 701 | 2.73 | 34.6 | 11.9 | 4.5 | 121 |
| Example 1-1 | 724 | 2.23 | 31.1 | 11.3 | 2.2 | 125 |
| Example 1-2 | 733 | 2.28 | 32.1 | 12.5 | 2.3 | 122 |
| Comparative example 1-3 | 739 | 2.24 | 31.8 | 13.5 | 2.5 | 121 |
| Example a | 720 | 2.52 | 32.8 | 12.3 | 3.9 | 122 |
| Example b | 699 | 2.49 | 32.5 | 12.7 | 3.3 | 126 |
| Example c | 729 | 2.25 | 31.5 | 13.1 | 2.2 | 127 |

| | Dirt levels | | | | | |
|---|---|---|---|---|---|---|
| | Enzymatic assay | | | Staining assay (60 g/m2 handsheet) | | Dirt assay (60 g/m2 handsheet) | |
| | Weight (%) | | | Number of particles | Area | Number of particles | Area |
| | Pulp | SAP | Nonwovens, plastics | Counts/m2 | mm2/m2 | Counts/m2 | mm2/m2 |
| Comparative example 1-1 | 58.4 | 13.5 | 28.1 | 160,000 | 68,000 | 2,100 | 291 |
| Comparative example 1-2 | 100.0 | - | - | 100 | 5 | 33 | 5 |
| Example 1-1 | 91.6 | 0.3 | 8.1 | 4,400 | 440 | 340 | 37 |
| Example 1-2 | 89.3 | 2.3 | 8.4 | 30,000 | 5,400 | 600 | 68 |
| Comparative example 1-3 | 85.9 | 6.1 | 8.0 | 90,000 | 33,000 | 1,100 | 180 |
| Example a | 92.2 | 0.2 | 7.6 | 180 | 17 | 42 | 4 |
| Example b | 91.6 | 0.1 | 8.3 | 360 | 44 | 98 | 11 |
| Example c | 91.9 | 0.2 | 7.9 | 1,200 | 112 | 360 | 39 |

| | Handsheet | | | | | | | | | | | Appearance evaluation |
|---|---|---|---|---|---|---|---|---|---|---|---|---|
| | Basis weight (g/m2) | Density (g/m3) | Brightness (%) | Opacity (%) | Formation (%) | Breaking length (km) | Young's modulus (GPa) | Bending stiffness (μN.m) | Burst index (kPa/g/m2) | Tear index (mN/g/m2) | Rate of change in hygroscopicity (%) | |
| Comparative example 1-1 | 68.5 | 0.47 | 80.9 | 70.1 | 5.7 | 1.8 | 1.1 | 136 | 1.3 | 11.6 | 14.2 | × |
| Comparative example 1-2 | 67.1 | 0.44 | 84.4 | 72.8 | 5.3 | 2.4 | 1.9 | 251 | 1.6 | 14.9 | 11.9 | ○ |
| Example 1-1 | 66.0 | 0.48 | 83.4 | 71.3 | 5.6 | 2.4 | 1.9 | 172 | 1.6 | 16.0 | 11.6 | ○ |
| Example 1-2 | 65.9 | 0.47 | 81.9 | 71.9 | 5.6 | 2.2 | 1.5 | 169 | 1.5 | 13.8 | 11.8 | △ |
| Comparative example 1-3 | 66.0 | 0.47 | 80.9 | 69.7 | 5.9 | 2.1 | 1.3 | 154 | 1.4 | 11.4 | 11.4 | △ |
| Example a | 67.1 | 0.44 | 85.9 | 72.5 | 5.3 | 2.3 | 1.8 | 242 | 1.4 | 15.4 | 11.6 | ○ |
| Example b | 66.6 | 0.44 | 85.3 | 72.6 | 5.5 | 2.4 | 1.9 | 228 | 1.5 | 16.2 | 11.9 | ○ |
| Example c | 66.3 | 0.47 | 84.2 | 72.2 | 5.5 | 2.3 | 1.8 | 187 | 1.5 | 14.4 | 11.5 | ○ |

**Claims**

1. A recycled fiber obtained by subjecting a sanitary product containing a cellulose pulp to a recycling process, which has a superabsorbent polymer (SAP) content of less than 5 %, a Canadian standard freeness of 650 ml or more when determined according to JIS P 8121:1995, a length weighted average fiber length of 0.7 mm or more, and a dirt area fraction of 200 $mm^2/m^2$ or less, when determined by a dirt assay on a handsheet having a basis weight of 60 $g/m^2$ prepared according to JIS P 8209.

2. The recycled fiber of claim 1, which has a dirt area fraction of 50,000 $mm^2/m^2$ or less when determined by a staining assay using cobalt (II) chloride hexahydrate on a handsheet having a basis weight of 60 $g/m^2$ prepared according to JIS P 8209.

3. The recycled fiber of claim 1 or 2, which has a superabsorbent polymer content of 1 % or less.

4. The recycled fiber of any one of claims 1 to 3, which has a dirt area fraction of 1,000 $mm^2/m^2$ or less when determined by a staining assay using cobalt (II) chloride hexahydrate on a handsheet having a basis weight of 60 $g/m^2$ prepared according to JIS P 8209.

5. The recycled fiber of any one of claims 1 to 4 for use in construction materials, paperboards, cushioning materials or molded products.

6. The recycled fiber of claim 3 or 4 for use in sanitary products, fluff pulps, writing and printing papers or tissue papers.

7. A slurry comprising the recycled fiber of any one of claims 1 to 6.

8. A wet pulp comprising the recycled fiber of any one of claims 1 to 6.

9. A dry pulp comprising the recycled fiber of any one of claims 1 to 6.

10. A recycled fiber molding comprising a recycled fiber of claim 1.

11. A process for preparing the recycled fiber of any one of claims 1 to 6, comprising at least the steps of disintegrating a used sanitary product and dispersing it in water, and separating and recovering fibers and SAPs contained in the sanitary product, **characterized in that** the step of disintegrating a sanitary product and dispersing it in water comprises adding a bleaching/microbicidal agent, a crosslinking agent and an acidic agent as additives;
wherein the step of disintegrating a sanitary product and dispersing it in water comprises disintegration at a consistency of 3 to 20 % in the presence of sodium hypochlorite as a bleaching/microbicidal agent and calcium chloride as a crosslinking agent, followed by dilution to a consistency of 2 % or less and then adding an acidic agent to adjust the pH at 4.5 to 5.5, and also comprises heating at one or more points of the step;
and wherein the separation/recovery step comprises using a cascade separator or feed forward separator having two or more stages.

**Patentansprüche**

1. Recycling-Faserstoff, der erzeugt wird, indem ein Sanitärerzeugnis, das einen Zellstoff enthält, einem Recycling-Prozess unterzogen wird, aufweisend einen Gehalt an superabsorbierendem Polymer (SAP) von weniger als 5 %, einen CSF-Mahlgrad von 650 ml oder mehr, wenn nach JIS P 8121:1995 bestimmt, eine längengewichtete mittlere Faserlänge von 0,7 mm oder mehr und einen Schmutzflächenanteil von 200 $mm^2/m^2$ oder weniger, wenn durch eine Schmutzprobe an einem nach JIS P 8209 hergestellten Prüfblatt (Handsheet) mit einer Grammatur von 60 $g/m^2$ bestimmt.

2. Recycling-Faserstoff nach Anspruch 1, der einen Schmutzflächenanteil von 50.000 $mm^2/m^2$ oder weniger aufweist, wenn durch eine Färbeprobe mit Hilfe von Cobalt(II)-chloridhexahydrat an einem nach JIS P 8209 hergestellten Prüfblatt mit einer Grammatur von 60 $g/m^2$ bestimmt.

3. Recycling-Faserstoff nach Anspruch 1 oder 2, der einen Gehalt an superabsorbierendem Polymer von 1 % oder weniger aufweist.

**4.** Recycling-Faserstoff nach einem der Ansprüche 1 bis 3, der einen Schmutzflächenanteil von 1.000 mm$^2$/m$^2$ oder weniger aufweist, wenn durch eine Färbeprobe mit Hilfe von Cobalt(II)-chloridhexahydrat an einem nach JIS P 8209 hergestellten Prüfblatt mit einer Grammatur von 60 g/m$^2$ bestimmt.

**5.** Recycling-Faserstoff nach einem der Ansprüche 1 bis 4 zur Verwendung in Baustoffen, Pappen, Polstermaterialien oder Formprodukten.

**6.** Recycling-Faserstoff nach Anspruch 3 oder 4 zur Verwendung in Sanitärerzeugnissen, Flockenzellstoffen, Schreib- und Druckpapieren oder Tissue-Papieren.

**7.** Suspension, die den Recycling-Faserstoff nach einem der Ansprüche 1 bis 6 umfasst.

**8.** Nasser Halbstoff, der den Recycling-Faserstoff nach einem der Ansprüche 1 bis 6 umfasst.

**9.** Trockener Halbstoff, der den Recycling-Faserstoff nach einem der Ansprüche 1 bis 6 umfasst.

**10.** Recyclingfaserstoff-Formartikel, der einen Recycling-Faserstoff nach Anspruch 1 umfasst.

**11.** Verfahren zur Herstellung des Recycling-Faserstoffs nach einem der Ansprüche 1 bis 6, umfassend mindestens die Schritte: Zerfasern eines gebrauchten Sanitärerzeugnisses und dessen Dispergieren in Wasser, und Abscheiden und Wiedergewinnen von im Sanitärerzeugnis enthaltenen Fasern und SAPs, **dadurch gekennzeichnet, dass** der Schritt Zerfasern eines Sanitärerzeugnisses und dessen Dispergieren in Wasser umfasst: Zusetzen eines Bleichmittels/Mikrobizids, eines Vernetzungsmittels und eines Säuremittels als Additive; wobei der Schritt Zerfasern eines Sanitärerzeugnisses und dessen Dispergieren in Wasser umfasst: Zerfasern bei einer Stoffdichte von 3 bis 20 % in Gegenwart von Natriumhypochlorit als Bleichmittel/Mikrobizid und Calciumchlorid als Vernetzungsmittel, gefolgt vom Verdünnen auf eine Stoffdichte von 2 % oder weniger und dann Zusetzen eines Säuremittels zum Einstellen des pH-Werts auf 4,5 bis 5,5, und auch umfasst: Erwärmen an einer oder mehreren Stellen des Schritts; und wobei der Abscheidungs-/Rückgewinnungsschritt umfasst: Verwenden eines Kaskadenabscheiders oder Feedforward-Abscheiders mit zwei oder mehr Stufen.

## Revendications

**1.** Fibre recyclée obtenue en soumettant un produit sanitaire contenant une pulpe de cellulose à un procédé de recyclage, laquelle a une teneur en polymère superabsorbant (PSA) inférieure à 5 %, un indice d'égouttage CSF (Canadian Standard Freeness) de 650 ml ou plus lorsque celui-ci est déterminé selon JIS P 8121:1995, une longueur moyenne de fibre pondérée en longueur de 0,7 mm ou plus, et une fraction correspondant à la surface des impuretés de 200 mm$^2$/m$^2$ ou moins, lorsque celle-ci est déterminée par un essai de détection d'impuretés sur une feuille d'essai ayant un grammage de 60 g/m$^2$ préparée selon JIS P 8209.

**2.** Fibre recyclée selon la revendication 1, laquelle a une fraction correspondant à la surface des impuretés de 50 000 mm$^2$/m$^2$ ou moins lorsque celle-ci est déterminée par un essai de coloration pour lequel on utilise du chlorure de cobalt (II) hexahydraté sur une feuille d'essai ayant un grammage de 60 g/m$^2$ préparée selon JIS P 8209.

**3.** Fibre recyclée selon la revendication 1 ou 2, laquelle a une teneur en polymère superabsorbant de 1 % ou moins.

**4.** Fibre recyclée selon l'une quelconque des revendications 1 à 3, laquelle a une fraction correspondant à la surface des impuretés de 1 000 mm$^2$/m$^2$ ou moins lorsque celle-ci est déterminée par un essai de coloration pour lequel on utilise du chlorure de cobalt (II) hexahydraté sur une feuille d'essai ayant un grammage de 60 g/m$^2$ préparée selon JIS P 8209.

**5.** Fibre recyclée selon l'une quelconque des revendications 1 à 4, destinée à une utilisation dans des matériaux de construction, des papiers cartonnés, des matériaux d'amortissement ou des produits moulés.

**6.** Fibre recyclée selon la revendication 3 ou 4, destinée à une utilisation dans des produits sanitaires, des pâtes défibrées, des papiers d'écriture et d'impression ou des papiers minces.

**7.** Suspension comprenant la fibre recyclée selon l'une quelconque des revendications 1 à 6.

**8.** Pulpe humide comprenant la fibre recyclée selon l'une quelconque des revendications 1 à 6.

**9.** Pulpe sèche comprenant la fibre recyclée selon l'une quelconque des revendications 1 à 6.

**10.** Moulage en fibres recyclées comprenant une fibre recyclée selon la revendication 1.

**11.** Procédé de préparation de la fibre recyclée selon l'une quelconque des revendications 1 à 6, comprenant au moins les étapes de désagrégation d'un produit sanitaire usagé et de dispersion de celui-ci dans de l'eau, et de séparation et de récupération des fibres et des PSA présents dans le produit sanitaire, **caractérisé en ce que** l'étape de désagrégation d'un produit sanitaire et de dispersion de celui-ci dans de l'eau comprend l'addition d'un agent de blanchiment/microbicide, d'un agent de réticulation et d'un agent acide comme additifs ;
dans lequel l'étape de désagrégation d'un produit sanitaire et de dispersion de celui-ci dans de l'eau comprend une désagrégation à une consistance de 3 à 20 % en présence d'hypochlorite de sodium utilisée comme agent de blanchiment/microbicide et de chlorure de calcium utilisé comme agent de réticulation, suivie d'une dilution à une consistance de 2 % ou moins, avec ensuite l'addition d'un agent acide pour ajuster le pH jusqu'à l'obtention d'une valeur de 4,5 à 5,5, et comprend également un chauffage à un ou plusieurs points de l'étape ;
et dans lequel l'étape de séparation/récupération comprend l'utilisation d'un séparateur en cascade ou d'un séparateur à alimentation par devant comportant deux étages ou plus.

Figure 1

Figure 2

## REFERENCES CITED IN THE DESCRIPTION

**Patent documents cited in the description**

- JP 2000084533 A
- JP 2003135521 A
- JP 2003225645 A
- JP H6502454 A
- JP 2001310178 A
- JP 2003200147 A
- JP 2004042038 A
- JP 2004008270 A

- JP 2006007111 A
- EP 0558512 A1
- JP 2004082700 A
- US 2005220542 A1
- EP 1081285 A1
- JP 2012287476 A
- JP 2012287496 A
- JP 2009148441 A

**Non-patent literature cited in the description**

- *From Waste to Profitable Material; Recovery of Cellulose Fibers and Superabsorbents Improve Profitability,* http://www.venti-oelde.com/uploads/tx_sbdownloader/sap_rueckgewin-ed.pdf

- **IKEURA et al.** Quantification of pulp and polymer absorbents in recycling processes of paper diapers. *Journal of Environmental Laboratories Association,* 2011, vol. 36 (1), 51, , 58